# EUROPEAN PATENT SPECIFICATION

(11) **EP 3 358 001 B1**
(45) Date of publication and mention of the grant of the patent: **29.01.2020**
(21) Application number: 16850372.0
(22) Date of filing: 29.09.2016
(51) Int. Cl.: C12N 1/20, C12P 7/10, C12R 1/145

(54) **CLOSTRIDIUM BEIJERINCKII, USE THEREOF, AND METHOD OF PRODUCING BUTANOL**
CLOSTRIDIUM BEIJERINCKII, VERWENDUNG DAVON UND VERFAHREN ZUR HERSTELLUNG VON BUTANOL
CLOSTRIDIUM BEIJERINCKII, SON UTILISATION, ET PROCÉDÉ DE PRODUCTION DE BUTANOL

(30) Priority: 30.09.2015 CN 201510638879
(43) Date of publication of application: 08.08.2018
(73) Proprietor: China Petroleum & Chemical Corporation, Beijing 100728 (CN); Fushun Research Institute of Petroleum and Petrochemicals, Sinopec Corp., Fushun, Liaoning 113001 (CN)
(72) Inventor: ZHANG, Quan, Fushun Liaoning 113001 (CN); CAO, Changhai, Fushun Liaoning 113001 (CN); GAO, Huipeng, Fushun Liaoning 113001 (CN); GUAN, Hao, Fushun Liaoning 113001 (CN); WANG, Lingmin, Fushun Liaoning 113001 (CN); QIAO, Kai, Fushun Liaoning 113001 (CN)
(74) Representative: Hoffmann Eitle
(86) International application number: PCT/CN2016/100764
(87) International publication number: WO 2017/054748

(56) References cited:
- EP-A1- 2 182 051
- CN-A- 102 533 612
- CN-A- 102 618 479
- CN-A- 102 719 371
- CN-A- 103 820 367
- JP-A- 2009 039 031
- QURESHI N ET AL: "Butanol production by Clostridium beijerinckii. Part I: Use of acid and enzyme hydrolyzed corn fiber", BIORESOURCE TECHNOLOGY, ELSEVIER, AMSTERDAM, NL, vol. 99, no. 13, 1 September 2008 (2008-09-01), pages 5915-5922, XP022647443, ISSN: 0960-8524, DOI: 10.1016/J.BIORTECH.2007.09.087 [retrieved on 2007-12-03]

### SEQUENCE LISTING

<110> China Petroleum & chemical Corporation; Fushun Research Institute of Petroleum and Petrochemicals, SINOPEC CORP.
<120> CLOSTRIDIUM BEIJERINCKII, USE THEREOF, AND METHOD OF PRODUCING BUTANOL
<130> PCT40578SINO
<150> CN201510638879.7
   <151> 2015-09-30
<160> 1
<170> PatentIn version 3.5
<210> 1
   <211> 1299
   <212> DNA
   <213> Clostridium beijerinckii
<400> 1
   caactctcat ggtgtgacgg gcggtgtgta caaggcccgg gaacgtattc accgcgacat        60
   tctgattcgc gattactagc aactccagct tcatgtaggc gagtttcagc ctacaatccg       120
   aactgagact ggttttaaag tttggctcca cctcacggtt tagcatctct ctgtaccagc       180
   cattgtagca cgtgtgtagc cctagacata aggggcatga tgatttgacg tcatccccac       240
   cttcctcccg gttaacccgg gcagtctcgc tagagtgctc aactaaatgg tagcaactaa       300
   caataagggt tgcgctcgtt gcgggactta acccaacatc tcacgacacg agctgacgac       360
   aaccatgcac cacctgtctt cctgccccga agggcttccc cgattaaggg taattcagga       420
   gatgtcaagt ctaggtaagg ttcttcgcgt tgcttcgaat taaaccacat gctccgctgc       480
   ttgtgcgggc ccccgtcaat tcctttgagt tttaatcttg cgaccgtact ccccaggcgg       540
   aatacttaat gcgttagcgg cggcacagag gtcatgacaa cccctacacc tagtattcat       600
   cgtttacggc gtggactacc agggtatcta atcctgtttg ctccccacgc tttcgagcct       660
   cagtgtcagt tacagtccag aaagtcgcct tcgccactgg tattcttcct aatctctacg       720
   catttcaccg ctacactagg aattctactt tcctctcctg cactctagat atccagtttg       780
   gaatgcagca cccaggttaa gcccgagtat ttcacatccc acttaaatat ccacctacgc       840
   tccctttacg cccagtaaat ccggacaacg cttgctacct acgtattacc gcggctgctg       900
   gcacgtagtt agccgtggct tcctcctcag gtaccgtcat tatcgtccct gaagacagag       960
   ctttacaatc cgaagaccgt catcactcac gcggcgttgc tgcatcaggg tttcccccat      1020
   tgtgcaatat tccccactgc tgcctcccgt aggagtctgg gccgtgtctc agtcccaatg      1080
   tggccgatca ccctctcagg tcggctacgc atcgtcgcct tggtgagccg ttacctcacc      1140
   aactagctaa tgcgacgcgg gtccatctca tagcggatta ctcctttaat tgctatgcca      1200
   tgcggcacta caatcttatg cggtattaat cttcctttcg aaaggctatt cccctctatg      1260
   aggcaggtta cccacgtgtt actcacccgt ccgccgcta                              1299

## Description

### Field of the Invention

The present invention belongs to the technical field of microbes, in particular to a *Clostridium beijerinckii* that can be used to produce butanol, uses thereof, and a method for producing butanol by using the *C*. *beijerinckii.*

### Background of the Invention

Butanol is an important raw material in the organic chemical industry, and is widely applied in industrial sectors, including chemical industry, pharmaceutical, and petroleum sectors, etc. In addition, owing to the fact that butanol has two more methylene than ethanol, butanol has higher hydrophobicity and lower volatility, can be mixed with gasoline at any ratio, and has a heat value comparable to that of gasoline. As a renewable biological source that is a potential substitute for gasoline, butanol receives more and more attention in the countries around the world.

The butanol production processes mainly include chemical synthesis process and microbial fermentation process. As the petroleum resources are depleted increasingly, it is more and more difficult to produce butanol from petroleum through a method using propylene as raw material by hydroformylation. Moreover, for a long time, the supply of butanol in the market in China is inadequate and can't meet the demand owing to insufficient productive capacity as a result of out-of-date technology and small scale of installations. The microbial fermentation process for producing butanol shows unique advantages, and the present situation of inadequate supply of butanol will be alleviated greatly if bio-butanol techniques are developed.

The main products of acelone-butanol fermentation (ABF) are acetone, butanol and ethanol, at a concentration ratio of 3:6:1. All conventional acelone-butanol fermentation processes have the following problems: (1) the strains for conventional fermentation must be cultured and ferment under a strict anaerobic condition, air may invade and consequently the strains can't grow normally if there is any improper operation; therefore, usually an inert gas (e.g., N₂) has to be introduced in the fermentation process to maintain an oxygen-free environment, which result in a high energy consumption; (2) the yield ratio of butanol is low (only about 20wt%), thus the raw material cost in the butanol fermentation process is high, and the development of the butanol fermentation industry is limited; (3) the fermentation products include 40wt% byproducts (e.g., acetone and ethanol, etc.) besides butanol, and consequently the difficulty of product separation is increased, resulting in increased energy consumption.

Wherein, the problem of strains and raw material is always a bottleneck that hampers butanol fermentation. In recent years, many researches on butanol production through fermentation of fibrous raw materials have been made in different countries, mainly focusing on strain mutagenesis and breeding, seeking for proper fibrous raw materials and preparation of sugar solutions thereof, optimization of fermentation process, and solvent extraction, etc. At present, the strains for butanol production used in the industry are mainly *Clostridium acetobutylicum* and *C*. *beijerinckii,* which have a similar metabolism path, and the products are mainly categorized into three types: 1) solvents (acetone, ethanol and butanol); 2) organic acids (acetic acid, lactic acid and butanoic acid); 3) gasses (including carbon dioxide and hydrogen, etc.). The economic competitiveness of ABE (i.e., solvents) can be further improved by recycling the hydrogen in the products. However, the generation of the byproducts (e.g., acetone and ethanol, etc.) consumes the limited carbon metabolic flow, decreases the proportion of butanol in the products, and increases the difficulties in butanol recovery.

CN102162001A has provided a method for producing butanol through anaerobic fermentation utilizing *Clostridium acetobutylicum,* in which *C*. *acetobutylicum* XY16, *C*. *acetobutylicum* AS1.134, or *C*. *acetobutylicum* AS1.135 is used as a fermentation strain (strict anaerobe), glucose is used as a substrate, at 60g/L initial glucose concentration, the pH is regulated and controlled in the acid generation stage and alcohol generation stage of *C*. *acetobutylicum* while N₂ is introduced to maintain an strict anaerobic environment in the fermentation tank, the yields of total solvent and butanol are 19.20-19.65g/L and 11.43-12.30g/L respectively, the butanol selectivity is 58.2-63.1%, the yield ratio of the solvent is 32.0-32.8%, and the yield ratio of the butanol is 19.1-20.5%.

However, that method requires a strict anaerobic environment for fermentation and involves a risk that the strain can't grow normally because of incomplete deoxidization; in addition, the contents of byproducts (e.g., acetone and ethanol) are high in the fermentation products, and consequently the load of butanol recovery in the follow-up procedures is high, as well as the energy consumption. CN102719371A has disclosed a *C*. *beijerinckii* Y-3 for producing butanol, which is a mutant strain (strict anaerobe) obtained through mutagenesis of an original strain *C*. *beijerinckii* NCIMB 8052 with ethylmethane sulfonate (EMS), and can be used to produce bio-butanol from xylose cinder; when enzymatic hydrolysate of xylose cinder is used as a carbon source, the yield of total solvent is 16g/L, the yield of butanol is 8.2g/L, the content of acetone is 6.8g/L, and the content of ethanol is 1.0g/L.

Though the problem that the strains are not capable enough and the raw materials are insufficient for producing butanol through the conventional biological fermentation process is solved by utilizing the strain described above, the fermentation process has to be executed in a strict anaerobic environment too; in addition, some drawbacks, including high butanol recovery load in the follow-up procedure and high energy consumption, etc., still exist, because of high content of byproducts (e.g., acetone and ethanol, etc.) in the fermentation products.

### Summary of the Invention

To overcome the above-mentioned drawbacks in the prior art, the present invention provides a novel strain of *C*. *beijerinckii* that can be used to produce butanol, a use thereof, and a method for producing butanol utilizing the *C*. *beijerinckii.* This strain can grow and ferment to produce butanol under a facultative anaerobic condition, and the present invention has advantages including high butanol yield and yield ratio, and less byproducts (e.g., acetone and ethanol), etc.

To attain the object described above, through numerous experiments, the inventor of the present invention obtains a strain of *C*. *beijerinckii,* which has strong oxygen tolerance, can grow normally under a facultative anaerobic condition, and achieves high butanol yield and yield ratio, when it is used to produce butanol through fermentation; in addition, there is nearly no byproduct (e.g., acetone and ethanol, etc.) in the fermentation products.

Therefore, in a first aspect, the present invention provides a strain of *C*. *beijerinckii,* which has been deposited in China General Microbiological Culture Collection Center and numbered as CGMCC No. 9124.

In a second aspect, the present invention provides a use of the *C*. *beijerinckii* in butanol production. In a third aspect, the present invention provides a method for producing butanol, which comprises: inoculating the *C*. *beijerinckii* into a fermentation medium for fermenting cultivation to produce butanol.

The *C*. *beijerinckii* having deposit number CGMCC No. 9124 in the present invention belongs to facultative anaerobic bacteria, can grow normally under conditions of pH=4-9 and temperature = 20-42°C, and has strong oxygen tolerance in the fermentation process for butanol production.

Therefore, it is unnecessary to add any deoxidizer into the culture medium or introduce N₂ and/or any other inert gas to maintain an anaerobic environment during the entire process; thus, the deoxidization step in the conventional anaerobic fermentation process is omitted, a phenomenon that the strain can't grow normally as a result of incomplete deoxidization is avoided, and the energy consumption is reduced. In addition, through fermentation under a facultative anaerobic condition with the *C*. *beijerinckii* disclosed in the present invention, the butanol yield and yield ratio are high, and there is nearly no byproduct (e.g., acetone and ethanol, etc.) in the obtained fermentation products, the butanol recovery load in the follow-up procedures is alleviated, and the energy consumption for separation is reduced. Moreover, the *C*. *beijerinckii* can ferment under an anaerobic condition to produce butanol.

Other features and advantages of the present invention will be further detailed in the embodiments hereunder.

### Biological Deposition

The *C*. *beijerinckii* disclosed in the present invention was deposited in China General Microbiological Culture Collection Center (CGMCC, address: Institute of Microbiology, Chinese Academy of Sciences, Building 3, No.1 West Beichen Road, Chaoyang District, Beijing, postal code: 100101) on May 4, 2014 and numbered as CGMCC No. 9124.

### Description of the Embodiments

Hereunder some embodiments of the present invention will be detailed. It should be understood that the embodiments described here are only provided to describe and explain the present invention, but shall not be deemed as constituting any limitation to the present invention.

The ends points and any value in the ranges disclosed in the present invention are not limited to the exact ranges or values; instead, those ranges or values shall be comprehended as encompassing values that are close to those ranges or values. For numeric ranges, any values between the end points of the ranges, the end points of the ranges and the discrete point values, and the discrete point values may be combined to obtain one or more new numeric ranges, which shall be deemed as having been disclosed specifically in this document.

In a first aspect, the present invention provides a strain of *C*. *beijerinckii,* having deposit number CGMCC No. 9124.

The *C*. *beijerinckii* described in the present invention belongs to gram-positive bacteria, with rod-shaped cell morphology, and can generate spore. It is identified through physiological and biochemical identification as catalase negative, oxidase negative, and nitrate inassimilable. The specific physiological and biochemical characteristics are shown in Table 1.

**Table 1**

| Test item | Result | Test item | Result | Test item | Result |
|---|---|---|---|---|---|
| Cell morphology | Rod shape | Gram staining | Positive | Sporulation | + |
| Grow at 15 °C | - | Catalase | - | Oxidase | - |
| 4% NaCl | - | pH5.0 | + | Nitrate reduction | - |

| API 50CH | | | | | |
|---|---|---|---|---|---|
| Glycerol | - | Inositol | - | Inulin | - |
| Erythritol | - | Mannitol | - | Melizitose | - |
| D-arabinose | - | Sorbitol | - | Raffinose | + |
| L-arabinose | - | α-methyl-D-mannoside | - | Starch | + |
| D-ribose | + | α-methyl-D-glycoside | + | Glycogen | + |
| D-xylose | - | N-acetyl-glucosamine | + | Xylitol | - |
| L-xylose | + | Amygdalin | + | Gentiobiose | + |
| Adonitol | - | Arbutin | + | D-turanose | + |
| β-methyl-D-xyloside | - | Esculin | + | D-lyxose | - |
| D-galactose | + | Salicin | + | D-tagatose | - |
| D-glucose | + | Cellobiose | + | D-fucose | - |
| D-fructose | + | Maltose | + | L-fucose | - |
| D-mannose | + | Lactose | - | D-arabitol | - |
| L-sorbose | - | Melibioze | - | L-arabitol | - |
| L-rhamnose | - | Sucrose | + | Gluconate | - |
| Galactitol | - | Trehalose | + | 2-keto-gluconate | - |

| | | | | | |
|---|---|---|---|---|---|
| Note: "+" represents positive reaction or usable; "-" represents negative reaction or unusable | | | | | |

Total DNA of strain is extracted from the *C*. *beijerinckii,* and 16S rRNA sequence analysis is carried out. The 16S rDNA sequence is shown by SEQ ID NO: 1. The *C*. *beijerinckii* is named as XH0906, deposited in China General Microbiological Culture Collection Center, and numbered as CGMCC No. 9124.

The *C*. *beijerinckii* described in the present invention belongs to facultative aerobe (i.e., facultative anaerobe), and can grow well under conditions of pH=4-9 and temperature = 20-42 °C. When it is cultured and ferments under a facultative anaerobic condition, butanol can be obtained at a high yield ratio, and there is nearly no acetone and ethanol in the fermentation products; in addition, the *C*. *beijerinckii* can ferment under an anaerobic condition (strict anaerobic condition) to produce butanol.

In a second aspect, the present invention provides a use of the *C*. *beijerinckii* in butanol production, especially in butanol production through fermentation under a facultative anaerobic condition.

In a third aspect, the present invention provides a method for producing butanol, which comprises: inoculating the strain of *C*. *beijerinckii* disclosed in the present invention into a fermentation medium for fermentation to produce butanol.

In the butanol production method disclosed in the present invention, the fermentation may be executed under an anaerobic condition (strict anaerobic condition) or facultative anaerobic condition, preferably under a facultative anaerobic condition.

In the butanol production method disclosed in the present invention, preferably, the facultative anaerobic condition is created by a method including: no deoxidizer is added into the culture medium, and no N₂ and/or inert gas is introduced to maintain an anaerobic environment during the cultivation. That is to say, in the present invention, the facultative anaerobic condition means that it is unnecessary to add any deoxidizer (e.g., sodium hydrosulfite, etc.) into the culture medium or introduce N₂ and/or inert gas to maintain an anaerobic environment during the cultivation (including seed cultivation and fermentation). Generally, the change of partial pressure of oxygen in the cultivation environment under the facultative anaerobic condition is as follows: the pressure in the fermentation tank is atmospheric pressure in the fermentation process; the partial pressure of oxygen in the fermentation tank is 20%±1% in the initial fermentation state, drops and may drop to 0 since large quantities of CO₂ and hydrogen are generated as the fermentation proceeds, and rises as the generated gasses are reduced in the late stage of fermentation.

In the butanol production method disclosed in the present invention, preferably, the conditions of the fermentation include: fermentation temperature: 20-42 °C, more preferably 28-42 °C, most preferably 32-38°C.

Preferably, the conditions of the fermentation further include: fermentation time: 48-120h, more preferably 60-80h. The specific fermentation time may be determined according to the specific fermentation conditions.

Preferably, the fermentation is performed under stirring condition, and more preferably, the stirring rate is 50-200rpm.

Preferably, the conditions of the fermentation further include: pH: 4-9, more preferably 5-7.

Wherein the initial pH of the fermentation medium is 5.5-8.5, preferably is 6-8. Furthermore, the inventor of the present invention has found during the research: in the fermentation process, the pH of the fermentation broth drops firstly (may drop to 4-5) and then rises as the fermentation proceeds.

After the pH of the fermentation broth drops from the initial pH of the culture medium to any value within a range of 5.5-6.5, the pH of the fermentation broth is controlled to remain within the range of 5.5-6.5 until the end of fermentation, thus the yield and yield ratio of butanol can be further improved. Therefore, to further improve the yield and yield ratio of butanol, preferably, during the fermentation process, after the pH of the fermentation broth drops to any value within the range of 5.5-6.5, the pH of the fermentation broth is controlled to be 5.5-6.5 (within this range or at a specific value within this range) until the end of fermentation.

According to a preferred embodiment of the present invention, the conditions of the fermentation include: fermentation temperature: 28-42°C, more preferably 34-38°C; the pH is a natural value in the initial fermentation stage; and is controlled to 4-7, more preferably 5-6, after logarithmic phase; fermentation time: 72-120h. Generally, the *C*. *beijerinckii* disclosed in the present invention can enter into the logarithmic phase after it is cultivated under above-mentioned fermentation conditions for 12-36h. Wherein, the logarithmic phase may be judged by detecting OD₆₀₀ value of the fermentation broth (i.e., light absorption value at 600nm wavelength), and usually the OD₆₀₀ value in logarithmic phase is 1-5.

In the butanol production method disclosed in the present invention, there is no particular restriction on the specific fermentation process, as long as the conditions of fermentation described above are met. In other words, the fermentation process may be any fermentation process commonly used in the art, such as batch fermentation, batch-fed fermentation, continuous fermentation, in-situ extractive fermentation, or in-situ gas extractive fermentation, etc. The specific operation steps of each process are well known to those skilled in the art and will not be detailed further here.

The butanol production method disclosed in the present invention further comprises: performing strain activating cultivation and seed cultivation successively before the fermentation. The activating cultivation is to inoculate the strain in deposited state into a suitable culture medium and cultivate therein, to recover the fermentability of the strain. The purpose of the seed cultivation is to obtain pure and vigorous cultures, i.e., obtain cultures of the fermentative bacteria that is vigorous and in a quantity enough for inoculation. Wherein, there is no particular restriction on the strain activating cultivation and seed cultivation. In other words, the strain activating cultivation and seed cultivation may be performed under different cultivation conditions commonly used in the art.

Preferably, the strain activating cultivation in the present invention is performed under an anaerobic condition, and the seed cultivation is performed under a facultative anaerobic condition.

Preferably, the method for producing butanol comprises:
(1) inoculating the *C*. *beijerinckii* into a solid culture medium, and culturing at 28-42°C for 12-48h under an anaerobic condition;
(2) inoculating the *C*. *beijerinckii* cultured in step (1) into a seed culture medium, and statically culturing at 28-42°C under a facultative anaerobic condition for 12-48h, to obtain a seed solution;
(3) inoculating the seed solution obtained in step (2) in an inoculum size of 2-20vol% into a fermentation medium, and performing the fermentation under a facultative anaerobic condition. The specific conditions of fermentation have been described above, and will not be detailed repetitively.

In the butanol production method disclosed in the present invention, preferably, the fermentation medium comprises carbon source, nitrogen source, inorganic salt and vitamin, and the initial pH of the fermentation medium is 5.5-8.5, more preferably 6-8.

There is no particular restriction on the carbon source. In other words, the carbon source may be any carbon source commonly used in the art, such as at least one of saccharide, starch and lignocellulose raw materials, etc.; preferably, the carbon source is one or more of glucose, xylose, galactose, mannose, fructose, maltose, cellobiose, glucose oligosaccharide, and xylo-oligosaccharide. To further improve the yield and yield ratio of the butanol, more preferably, the carbon source is glucose and/or xylose, most preferably glucose.

There is no particular restriction on the nitrogen source. In other words, the nitrogen source may be any nitrogen source commonly used in the art; preferably, the nitrogen source includes an organic nitrogen source and an inorganic nitrogen source, wherein, the organic nitrogen source is one or more of beef extract, yeast extract, peptone, corn steep liquor and soybean meal hydrolysate; to further improve the yield and yield ratio of the butanol, more preferably, the organic nitrogen source is one or more of soybean meal hydrolysate, peptone and beef extract, most preferably is peptone and beef extract. Preferably, the inorganic nitrogen source is one or more of ammonium acetate, sodium nitrate, and ammonium sulfate; to further improve the yield and yield ratio of the butanol, more preferably, the inorganic nitrogen source is ammonium sulfate and/or ammonium acetate, most preferably ammonium sulfate.

There is no particular restriction on the inorganic salt. In other words, the inorganic salt may be any inorganic salt commonly used in the art; preferably, the inorganic salt is one or more of dipotassium hydrogen phosphate, potassium dihydrogen phosphate, sodium hydrogen phosphate, sodium dihydrogen phosphate, sodium chloride, ferrous sulfate, ferric sulfate, magnesium sulfate, calcium chloride, and manganese sulfate. Wherein, the introduction of the phosphate can attains an effect of buffering the pH.

Preferably, the vitamin is one or more of vitamin B1, biotin, and para-aminobenzoic acid.

Preferably, on the basis of 1L fermentation medium, the dose of the carbon source is 20-60g, more preferably 40-50g; the dose of the organic nitrogen source is 1-20g, more preferably 10-18g; the dose of the inorganic nitrogen source is 0.1-10g, more preferably 0.5-5g; the dose of the inorganic salt is 0-10g, more preferably 2.5-5g; the dose of the vitamin is 0-0.2g, more preferably 0.04-0.12g.

The inventor of the present invention has further found through numerous experiments: on the basis of 1L fermentation medium, if the culture medium contains 40-50g glucose, 8-12g peptone, 4-8g beef extract, 0.6-1.2g ammonium sulfate, 0.25-0.75g sodium chloride, 0.05-0.2g ferric sulfate, 0.15-0.45g magnesium sulfate, 0.05-0.15g calcium chloride, 0.5-4g potassium dihydrogen phosphate, 1-4g sodium hydrogen phosphate, 0.01-0.06g para-aminobenzoic acid, 0.01-0.06g vitamin B1 and 0.001-0.006g biotin, the yield and yield ratio of the butanol can be further improved.

Therefore, preferably, on the basis of 1L fermentation medium, the fermentation medium contains 40-50g glucose, 8-12g peptone, 4-8g beef extract, 0.6-1.2g ammonium sulfate, 0.25-0.75g sodium chloride, 0.05-0.2g ferric sulfate, 0.15-0.45g magnesium sulfate, 0.05-0.15g calcium chloride, 0.5-4g potassium dihydrogen phosphate, 1-4g sodium hydrogen phosphate, 0.01-0.06g para-aminobenzoic acid, 0.01-0.06g vitamin B1, and 0.001-0.006g biotin.

In the butanol production method disclosed in the present invention, the composition of the seed culture medium may be the same as that of the fermentation medium, and a solid culture medium may be prepared by adding 1-2wt% agar to the liquid culture medium (e.g., the fermentation medium); however, the present invention is not limited to that. Those skilled in the art can appreciate: any other conventional culture medium that can be used for the activating cultivation and seed cultivation is within the scope of the present invention.

### Examples

Hereunder the present invention will be further detailed in examples, but the present invention is not limited to those examples.

Unless otherwise specified, the experimental methods in the following examples are conventional methods in the art. Unless otherwise specified, the experimental materials used in the following examples are obtained by purchasing from stores of conventional biochemical reagents.

The method for preparing soybean meal hydrolysate comprises: weighing soybean meal in an appropriate quantity, adding water in a quantity equal to 5 times of the mass of the soybean meal, and mixing the materials to a homogenous state; adding concentrated sulfuric acid at 98% mass concentration in volume equal to 2% of the volume of water, and mixing the materials into a homogeneous state quickly to avoid partial carbonization of the materials; introducing vapor to heat up to the mixture to 100°C, holding at the temperature for 20h while stirring for 5min per hour.

After the hydrolysis is finished, soybean meal hydrolysate in garnet-red color with a fruity smell is obtained.

The products and byproducts in the fermentation broth are analyzed with a liquid chromatograph, to calculate the concentrations of the main constituents. The liquid chromatograph is Agilent 1200, the chromatographic column is HPX-87H (300mmx7.8mm), the flowing phase is 0.005mol/L H₂SO₄ at 0.6ml/min flow rate, the column temperature is 65°C, the detector is a refractive index detector (Agilent 1200), the detector temperature is 45°C, and the sample size is 5µL.

Yield ratio of butanol = concentration of butanol obtained through fermentation / (concentration of glucose before fermentation - concentration of glucose after fermentation).

### Example 1

This example is provided to describe the method of producing butanol through fermentation under a facultative anaerobic condition with the *C*. *beijerinckii* having deposit number CGMCC No. 9124 in the present invention.

Composition of the liquid culture medium (including seed culture medium and fermentation medium): 10g/L peptone, 6g/L beef extract, 45g/L glucose, 0.5g/L sodium chloride, 0.9g/L ammonium sulfate, 0.1g/L ferric sulfate, 0.3g/L magnesium sulfate, 0.1g/L calcium chloride, 1g/L potassium dihydrogen phosphate, 2g/L sodium hydrogen phosphate, 0.04g/L para-aminobenzoic acid, 0.04g/L vitamin B1, and 0.004g/L biotin, pH=7.0, and the medium is sterilized at 121°C for 15min. The solid culture medium is prepared by adding 1wt% agar to the liquid culture medium.

The method for producing butanol through fermentation under a facultative anaerobic condition with the *C*. *beijerinckii* is as follows: (1) the *C*. *beijerinckii* is inoculated on a slant surface of the solid culture medium, and cultivated in an anaerobic environment at 30°C for 24h; (2) two rings of the *C*. *beijerinckii* cultivated in step (1) are scraped off from the slant surface and then inoculated into a seed culture medium disposed in a shaking flask, and cultivated in a static state at 35°C for 24h, without adding any deoxidizer or introducing N₂ to maintain an anaerobic environment; thus, a seed solution is obtained; (3) the seed solution cultivated in step (2) is inoculated in 10vol% inoculum size into 3L liquid fermentation medium disposed in a 5L fermentation tank, and fermentation cultivating at 37°C while it is stirred at 150r/min stirring rate, without adding any deoxidizer or introducing N₂ to maintain an anaerobic environment; as the fermentation proceeds, the pH of the fermentation broth drops firstly, and then rises; after the pH of the fermentation broth drops from the original pH=7.0 to pH=6.0, 10M NaOH solution is added by batch feeding, to control the pH of the fermentation broth at 6.0 until the end of the fermentation. As indicated by the detection result, after 72h fermentation, the concentration of residual glucose in the fermentation broth is 9.67g/L, the concentration of acetone is 0.04g/L, the concentration of ethanol is 0.02g/L, the concentrations of butanol, acetic acid, and butanoic acid are 10.17g/L, 0.56g/L, and 0.42g/L respectively, and the yield ratio of butanol is 28.79%.

### Example 2

The method described in example 1 is used, but the glucose, peptone or ammonium sulfate in the culture medium is replaced with other carbon source and nitrogen source respectively. As indicated in the detection result, after 72h fermentation, the concentration of acetone in the fermentation broth is lower than 0.1g/L, the concentration of ethanol is lower than 0.1g/L, the concentration of residual glucose (residual sugar) and the fermentation result of acetic acid, butanoic acid and butanol are shown in Table 2.

**Table 2**

| No. | Constituents of culture medium | | Fermentation result (g/L) | | | | |
|---|---|---|---|---|---|---|---|
| | Carbon source | Nitrogen source | Residual glucose | Acetic acid | Butanoic acid | Butanol | Yield ratio of butanol (%) |
| 1 | Xylose | The same as that in example 1 | 11.62 | 0.72 | 0.45 | 9.13 | 27.35 |
| 2 | Glucose : xylose (1:1, concentration ratio, g/L) | The same as that in example 1 | 11.35 | 0.64 | 0.33 | 9.21 | 27.37 |
| 3 | Galactose | The same as that in example 1 | 10.87 | 0.50 | 0.39 | 9.38 | 27.48 |
| 4 | The same as that in example 1 | Soybean meal hydrolysate | 9.98 | 0.57 | 0.57 | 9.60 | 27.41 |
| 5 | The same as that in example 1 | Ammonium acetate | 11.49 | 0.46 | 0.52 | 9.20 | 27.45 |

### Example 3

The method described in example 1 is used, but the composition of the liquid culture medium is: 10g/L peptone, 6g/L beef extract, 45g/L glucose, 0.5g/L sodium chloride, 0.9g/L ammonium sulfate, 0.1g/L ferric sulfate, 0.3g/L magnesium sulfate, 0.1g/L calcium chloride, 1g/L potassium dihydrogen phosphate, and 2g/L sodium hydrogen phosphate, pH=7.0.

As indicated in the detection result, after 72h fermentation, the concentration of residual glucose in the fermentation broth is 10.95g/L, the concentration of acetone is 0.09g/L, the concentration of ethanol is 0.05g/L, the concentrations of butanol, acetic acid and butanoic acid are 9.33g/L, 0.55g/L and 0.47g/L respectively, and the yield ratio of butanol is 27.4%.

### Example 4

The method described in example 1 is used, but the pH is not controlled in the entire fermentation process in step (3); as the fermentation proceeds, the pH of the fermentation broth drops firstly, and then rises, and the lowest pH is 4.5 in the fermentation process. As indicated in the detection result, after 72h fermentation, the concentration of residual glucose in the fermentation broth is 13.36g/L, the concentration of acetone is 0.05g/L, the concentration of ethanol is 0.03g/L, the concentrations of butanol, acetic acid and butanoic acid are 8.61g/L, 0.496g/L and 0.326g/L respectively, and the yield ratio of butanol is 27.21 %.

### Example 5

The method described in example 1 is used, but after the pH of the fermentation broth drops from original pH=7.0 to pH=5.5 in step (3), the pH of the fermentation broth is controlled to 5.5 until the end of the fermentation by adding 10M NaOH solution through batch feeding. As indicated in the detection result, after 72h fermentation, the concentration of residual glucose in the fermentation broth is 9.55g/L, the concentration of acetone is 0.05g/L, the concentration of ethanol is 0.03g/L, the concentrations of butanol, acetic acid and butanoic acid are 9.92g/L, 0.54g/L and 0.32g/L respectively, and the yield ratio of butanol is 27.98%.

### Example 6

The method described in example 1 is used, but after the pH of the fermentation broth drops from original pH=7.0 to pH=6.5 in step (3), the pH of the fermentation broth is controlled to 6.5 until the end of the fermentation by adding 10M NaOH solution through batch feeding. As indicated in the detection result, after 72h fermentation, the concentration of residual glucose in the fermentation broth is 10.41g/L, the concentration of acetone is 0.07g/L, the concentration of ethanol is 0.04g/L, the concentrations of butanol, acetic acid and butanoic acid are 9.67g/L, 0.54g/L and 0.9g/L respectively, and the yield ratio of butanol is 27.96%.

### Example 7

The method described in example 1 is used, but after the pH of the fermentation broth drops from the pH=7.0 to pH=5 in step (3), the pH of the fermentation broth is controlled to 5 until the end of the fermentation by adding 10M NaOH solution through batch feeding. As indicated in the detection result, after 72h fermentation, the concentration of residual glucose in the fermentation broth is 11.15g/L, the concentration of acetone is 0.08g/L, the concentration of ethanol is 0.04g/L, the concentrations of butanol, acetic acid and butanoic acid are 8.54g/L, 0.51g/L and 0.23g/L respectively, and the yield ratio of butanol is 25.23%.

### Example 8

The method described in example 1 is used, but a hermetically sealed anaerobic bottle is used during the seed cultivation in step (2), the culture medium is deoxidized in an anaerobic chamber, and sodium hydrosulfite is added into the anaerobic bottle so that the partial pressure of oxygen in the anaerobic bottle drops to 0, and then inoculation and cultivation are carried out in the anaerobic chamber; in step (3), sodium hydrosulfite is added to the culture medium in the fermentation tank for deoxidization, N₂ is introduced in the entire process to maintain an oxygen-free environment, and the pH is not controlled in the entire fermentation process. As indicated in the detection result, after 72h fermentation, the concentration of residual glucose in the fermentation broth is 5.0g/L, the concentration of acetone is 0.21g/L, the concentration of ethanol is 0.07g/L, the concentrations of butanol, acetic acid and butanoic acid are 9.98g/L, 1.22g/L and 1.46g/L respectively, and the yield ratio of butanol is 24.95%.

### Example 9

Composition of the liquid culture medium: 10g/L peptone, 6g/L beef extract, 40g/L glucose, 0.5g/L sodium chloride, 0.9g/L ammonium sulfate, 0.1g/L ferric sulfate, 0.3g/L magnesium sulfate, and 0.1g/L calcium chloride, pH=7.0, the liquid culture medium is sterilized at 121°C for 15min. The solid culture medium is prepared by adding 1wt% agar to the liquid culture medium.

The method for producing butanol through fermentation under a facultative anaerobic condition with the *C*. *beijerinckii* having deposit number CGMCC No. 9124 in the present invention is as follows: (1) the *C*. *beijerinckii* is inoculated on a slant surface of the solid culture medium, and cultivated in an anaerobic environment at 30°C for 24h; (2) two rings of the *C*. *beijerinckii* cultivated in step (1) are scraped off from the slant surface and then inoculated into a seed culture medium disposed in a shaking flask, and cultivated in a static state at 35°C for 24h, without adding any deoxidizer or introducing N₂ to maintain an anaerobic environment; thus, a seed solution is obtained; (3) the seed solution cultivated in step (2) is inoculated in 10vol% inoculum size into the liquid fermentation medium and cultivated without adding any deoxidizer or introducing N₂ to maintain an anaerobic environment, the natural pH is kept, and fermentation is carried out at 37°C for 88h. After 88h fermentation, the concentration of butanol in the fermentation broth is 7.5g/L, there is no acetone or ethanol product in the fermentation broth, and the concentrations of acetic acid and butanoic acid are 1.0g/L and 0.6g/L respectively.

### Example 10

The method described in example 9 is used, but the glucose, peptone or ammonium sulfate in the culture medium is replaced with other carbon source and nitrogen source respectively. The fermentation result is shown in Table 3.

**Table 3**

| No. | Conditions of fermentation | | Fermentation result (g/L) | | |
|---|---|---|---|---|---|
| | Carbon source | Nitrogen source | Acetic acid | Butanoic acid | Butanol |
| 1 | Xylose | The same as that in example 9 | 0.9 | 0.7 | 7.8 |
| 2 | Glucose : xylose (1:1, concentration ratio, g/L) | The same as that in example 9 | 1.1 | 0.5 | 7.4 |
| 3 | Galactose | The same as that in example 9 | 0.8 | 0.6 | 7.6 |
| 4 | The same as that in example 9 | Peptone is replaced with soybean meal hydrolysate | 0.7 | 0.9 | 7.4 |
| 5 | The same as that in example 9 | Ammonium sulfate is replaced with ammonium acetate | 1.2 | 0.8 | 7.3 |

### Example 11

A 5L fermentation tank is used for fermentation, glucose is used as a substrate, and the concentration of glucose in the initial culture medium is 40g/L. 0.1% potassium dihydrogen phosphate (1g/L), 0.2% sodium hydrogen phosphate (2g/L), 0.004% para-aminobenzoic acid (0.04g/L), 0.004% vitamin B1 (0.04g/L), and 0.0004% biotin (0.004g/L) are added on the basis of the composition of fermentation medium described in example 9. 3L liquid is loaded, the stirring rate is 150r/min., the pH is not controlled within the first 16h of fermentation (i.e., the pH drops naturally), and the pH is adjusted to 6.0 by adding 10M NaOH solution through batch feeding after 16h; the glucose is fully consumed in 72h. As indicated in the detection result, the concentrations of butanol, acetic acid and butanoic acid in the fermentation broth are 8.25g/L, 1.2g/L and 0.8g/L respectively.

It can be seen from the result of comparison between example 1 and example 2: in the process of producing butanol through fermentation under a facultative anaerobic condition with the *C*. *beijerinckii* having deposit number CGMCC No. 9124 in the present invention, if the carbon source in the culture medium is glucose, the organic nitrogen source is peptone and beef extract, and the inorganic nitrogen source is ammonium sulfate, the yield and yield ratio of butanol can be further improved.
It can be seen from the result of comparison between example 1 and example 3: in the process of producing butanol through fermentation under a facultative anaerobic condition with the *C*. *beijerinckii* having deposit number CGMCC No. 9124 in the present invention, if the culture medium further contains specific types of vitamins, the yield and yield ratio of butanol can be further improved.
It can be seen from the result of comparison between example 1 and examples 4-7: in the process of producing butanol through fermentation under a facultative anaerobic condition with the *C*. *beijerinckii* having deposit number CGMCC No. 9124 in the present invention, after the pH of the fermentation broth drops to any value with a range of 5.5-6.5 as the fermentation proceeds, if the pH of the fermentation broth is controlled to be 5.5-6.5 until the end of the fermentation, the yield and yield ratio of butanol can be further improved.
It can be seen from the result of example 8: even if the *C*. *beijerinckii* having deposit number CGMCC No. 9124 in the present invention is utilized to produce butanol through fermentation under an anaerobic condition, high yield and yield ratio of butanol still can be achieved.

In summary, the *C*. *beijerinckii* disclosed in the present invention, which has deposit number CGMCC No. 9124, can grow normally and ferment under a facultative anaerobic condition to produce butanol, and has strong oxygen tolerance. Therefore, it is unnecessary to add any deoxidizer into the culture medium or introduce any inert gas (e.g., N₂) to maintain an anaerobic environment in the entire process; thus, the deoxidization step in the conventional anaerobic fermentation process is omitted, a phenomenon that the strain can't grow normally as a result of incomplete deoxidization is avoided, and the energy consumption is reduced. In addition, through fermentation with the *C*. *beijerinckii* disclosed in the present invention, the butanol yield and yield ratio are high, and there is nearly no byproduct (e.g., acetone and ethanol, etc.) in the obtained fermentation products, the butanol recovery load in the follow-up procedures is alleviated, and the energy consumption for separation is reduced.

## Claims

1. A *Clostridium beijerinckii,* having deposit number CGMCC No. 9124.

2. Use of the *C*. *beijerinckii* according to claim 1 in producing butanol.

3. A method for producing butanol comprising: inoculating the *C*. *beijerinckii* according to claim 1 into a fermentation medium for fermentation to produce butanol.

4. The method according to claim 3, wherein the fermentation is executed under a facultative anaerobic condition.

5. The method according to claim 3 or 4, wherein the conditions of fermentation include: fermentation temperature: 20-42 °C , preferably 28-42 °C; fermentation time: 48-120h, preferably 60-80h; pH: 4-9, preferably 5-7.

6. The method according to claim 5, wherein the initial pH of the fermentation medium is 5.5-8.5, preferably 6-8; during the fermentation process, after the pH of the fermentation broth drops to any value within a range of 5.5-6.5, the pH of the fermentation broth is controlled to remain within the range of 5.5-6.5 until the end of fermentation.

7. The method according to any of claims 3-6, wherein the fermentation medium comprises carbon source, nitrogen source, inorganic salt and vitamin, and the initial pH of the fermentation medium is 5.5-8.5, preferably 6-8.

8. The method according to claim 7, wherein the carbon source is one or more of glucose, xylose, galactose, mannose, fructose, maltose, cellobiose, glucose oligosaccharide and xylo-oligosaccharide, preferably glucose and/or xylose;
the nitrogen source includes an organic nitrogen source and an inorganic nitrogen source, the organic nitrogen source is one or more of beef extract, yeast extract, peptone, corn steep liquor and soybean meal hydrolysate, preferably peptone and beef extract; the inorganic nitrogen source is one or more of ammonium acetate, sodium nitrate and ammonium sulfate, preferably ammonium sulfate;
the inorganic salt is one or more of dipotassium hydrogen phosphate, potassium dihydrogen phosphate, sodium hydrogen phosphate, sodium dihydrogen phosphate, sodium chloride, ferrous sulfate, ferric sulfate, magnesium sulfate, calcium chloride and manganese sulfate; the vitamin is one or more of vitamin B1, biotin and para-aminobenzoic acid;
preferably, on the basis of 1L fermentation medium, the dose of carbon source is 20-60g, more preferably 40-50g; the dose of organic nitrogen source is 1-20g, more preferably 10-18g; the dose of inorganic nitrogen source is 0.1-10g, more preferably 0.5-5g; the dose of the inorganic salt is 0-10g, more preferably 2.5-5g; the dose of vitamin is 0-0.2g, more preferably 0.04-0.12g.

9. The method according to claim 8, wherein on the basis of 1L fermentation medium, the fermentation medium contains 40-50g glucose, 8-12g peptone, 4-8g beef extract, 0.6-1.2g ammonium sulfate, 0.25-0.75g sodium chloride, 0.05-0.2g ferric sulfate, 0.15-0.45g magnesium sulfate, 0.05-0.15g calcium chloride, 0.5-4g potassium dihydrogen phosphate, 1-4g sodium hydrogen phosphate, 0.01-0.06g para-aminobenzoic acid, 0.01-0.06g vitamin B1, and 0.001-0.006g biotin.

10. The method according to any of claims 3-9, further comprising: performing strain activating cultivation and seed cultivation successively before fermentation,
preferably, the method for producing butanol comprises:
(1) inoculating the *C*. *beijerinckii* into a solid culture medium, and culturing at 28-42°C for 12-48h under an anaerobic condition;
(2) inoculating the *C*. *beijerinckii* cultured in step (1) into a seed culture medium, and statically culturing at 28-42°C under a facultative anaerobic condition for 12-48h, to obtain a seed solution;
(3) inoculating the seed solution obtained in step (2) in an inoculum size of 2-20vol% into a fermentation medium, and performing the fermentation under a facultative anaerobic condition.

11. The method according to claim 4 or 10, wherein the facultative anaerobic condition is created by a method including: no deoxidizer is added into the culture medium, and no N₂ and/or inert gas is introduced to maintain an anaerobic environment during the cultivation.

## Patentansprüche

1. *Clostridium beijerinckii,* mit der Hinterlegungsnummer CGMCC No. 9124.

2. Verwendung des *C*. *beijerinckii* gemäß Anspruch 1 zum Herstellen von Butanol.

3. Herstellungsverfahren für Butanol, umfassend:
inokulieren des *C*. *beijerinckii* gemäß Anspruch 1 in ein Fermentationsmedium für Fermentation, um Butanol herzustellen.

4. Verfahren gemäß Anspruch 3, wobei die Fermentation unter fakultativen anaeroben Bedingungen durchgeführt wird.

5. Verfahren gemäß Anspruch 3 oder 4, wobei die Bedingungen der Fermentation einschließen: Fermentationstemperatur: 20-42°C, bevorzugt 28-42°C; Fermentationszeit: 48-120h, bevorzugt 60-80h; pH: 4-9, bevorzugt 5-7.

6. Verfahren gemäß Anspruch 5, wobei der Anfangs-pH des Fermentationsmediums 5,5-8,5 ist, bevorzugt 6-8; während dem Fermentationsprozess, nachdem der pH der Fermentationsbrühe auf irgendeinem Wert innerhalb des Bereichs von 5,5-6,5 fällt, der pH der Fermentationsbrühe kontrolliert wird, um bis zum Ende der Fermentation innerhalb des Bereichs von 5,5-6,5 zu bleiben.

7. Verfahren gemäß irgendeinem der Ansprüche 3-6, wobei das Fermentationsmedium Kohlenstoffquelle, Stickstoffquelle, anorganisches Salz und Vitamin umfasst, und der Anfangs-pH des Fermentationsmedium 5,5-8,5 ist, bevorzugt 6-8.

8. Verfahren gemäß Anspruch 7, wobei die Kohlenstoffquelle eine oder mehrere von Glucose, Xylose, Galactose, Mannose, Fructose, Maltose, Cellobiose, Glucose-Oligosaccharid und Xylo-Oligosaccharid ist, bevorzugt Glucose und/oder Xylose;
die Stickstoffquelle eine organische Stickstoffquelle und eine anorganische Stickstoffquelle einschließt, die organische Stickstoffquelle eine oder mehrere von Rindfleischextrakt, Hefeextrakt, Pepton, Maisquellwasser und Sojabohnenmehl-Hydrolysat ist, bevorzugt Pepton und Rindfleischextrakt; die anorganische Stickstoffquelle eine oder mehrere von Ammoniumacetat, Natriumnitrat und Ammoniumsulfat ist, bevorzugt Ammoniumsulfat;
das anorganische Salz eins oder mehrere von Di-Kaliumhydrogenphosphat, Kaliumdihydrogenphosphat, Natriumhydrogenphosphat, Natriumdihydrogenphosphat, Natriumchlorid, Eisen(II)-sulfat ("ferrous sulfate"), Eisen(III)-Sulfat ("ferric sulfate"), Magnesiumsulfat, Calciumchlorid und Mangansulfat ist;
das Vitamin eins oder mehrere von Vitamin B1, Biotin und Para-Aminobenzoesäure ist;
bevorzugt, basierend auf 1L Fermentationsmedium, die Dosis Kohlenstoffquelle 20-60 g ist, bevorzugter 40-50g; die Dosis organische Stickstoffquelle 1-20g ist, bevorzugter 10-18g; die Dosis anorganische Stickstoffquelle 0,1-10g ist, bevorzugter 0,5-5g; die Dosis anorganisches Salz 0-10g ist, bevorzugter 2,5-5g; die Dosis Vitamin 0-0,2g ist, bevorzugter 0,04-0,12g.

9. Verfahren gemäß Anspruch 8, wobei, basierend auf 1L Fermentationsmedium, das Fermentationsmedium 40-50g Glucose, 8-12g Pepton, 4-8g Rindfleischextrakt, 0,6-1,2g Ammoniumsulfat, 0,25-0,75g Natriumchlorid, 0,05-0,2g Eisen(III)-Sulfat, 0,15g-0,45g Magnesiumsulfat, 0,05-0,15g Calciumchlorid, 0,5-4g Kaliumdihydrogenphosphat, 1-4g Natriumhydrogenphosphat, 0,01-0,06g Para-Aminobenzoesäure, 0,01-0,06g Vitamin B1 und 0,001-0,006g Biotin enthält.

10. Verfahren gemäß irgendeinem der Ansprüche 3-9, weiter umfassend: Durchführen einer Stamm-aktivierenden Kultivierung und anschließende Impf-Kultivierung vor der Fermentation,
bevorzugt umfasst das Verfahren zum Herstellen von Butanol:
(1) Inokulieren des *C*. *beijerinckii* in ein festes Kulturmedium, und Kultivieren bei 28-42°C für 12-48h unter anaeroben Bedingungen;
(2) Inokulieren des in Schritt (1) kultivierten *C*. *beijerinckii* in ein Impfkulturmedium, und statisches Kultivieren bei 28-42°C unter fakultativen anaeroben Bedingungen für 12-48h, um eine Impflösung zu erhalten;
(3) Inokulieren der in Schritt (2) erhaltenen Impflösung in einer Inokulum-Größe von 2-20 Volumen-% in ein Fermentationsmedium und Durchführen der Fermentation unter fakultativen anaeroben Bedingungen.

11. Verfahren gemäß Anspruch 4 oder 10, wobei die fakultative anaerobe Bedingung durch ein Verfahren erzeugt wird, einschließend: dem Kulturmedium wird kein Sauerstoff-entziehendes Mittel zugefügt und kein N₂ und/oder Inertgas wird eingeführt, um eine anaerobe Umgebung während der Kultivierung aufrechtzuerhalten.

## Revendications

1. *Clostridium beijerinckii,* présentant le numéro de dépôt CGMCC N° 9124.

2. Utilisation de la *C. beijerinckii* selon la revendication 1 dans la production de butanol.

3. Procédé de production de butanol comprenant : l'inoculation de *C*. *beijerinckii* selon la revendication 1 dans un milieu de fermentation pour fermentation afin de produire du butanol.

4. Procédé selon la revendication 3, dans lequel la fermentation est réalisée dans une condition anaérobie facultative.

5. Procédé selon la revendication 3 ou 4, dans lequel les conditions de fermentation incluent : température de fermentation : de 20 à 42 °C, de préférence de 28 à 42 °C ; temps de fermentation : de 48 à 120 h, de préférence de 60 à 80 h ; pH : de 4 à 9, de préférence de 5 à 7.

6. Procédé selon la revendication 5, dans lequel le pH initial du milieu de fermentation est compris entre 5,5 et 8,5, de préférence entre 6 et 8 ; pendant le processus de fermentation, une fois que le pH du bouillon de fermentation a chuté à une valeur quelconque dans une plage de 5,5 à 6,5, le pH du bouillon de fermentation est commandé pour rester dans la plage de 5,5 à 6,5 jusqu'à la fin de la fermentation.

7. Procédé selon l'une quelconque des revendications 3 à 6, dans lequel le milieu de fermentation comprend une source de carbone, une source d'azote, un sel inorganique et une vitamine, et le pH initial du milieu de fermentation est compris entre 5,5 et 8,5, de préférence entre 6 et 8.

8. Procédé selon la revendication 7, dans lequel la source de carbone est un ou plusieurs parmi le glucose, le xylose, le galactose, le mannose, le fructose, le maltose, le cellobiose, l'oligosaccharide de glucose et le xylo-oligosaccharide, de préférence le glucose et/ou le xylose ;
la source d'azote inclut une source d'azote organique et une source d'azote inorganique, la source d'azote organique est un ou plusieurs parmi un extrait de bœuf, un extrait de levure, une peptone, un extrait soluble de maïs et un hydrolysat de tourteau de soja, de préférence de la peptone et un extrait de bœuf ; la source d'azote inorganique est un ou plusieurs parmi l'acétate d'ammonium, le nitrate de sodium et le sulfate d'ammonium, de préférence le sulfate d'ammonium ;
le sel inorganique est un ou plusieurs parmi l'hydrogénophosphate de dipotassium, le dihydrogénophosphate de potassium, l'hydrogénophosphate de sodium, le dihydrogénophosphate de sodium, le chlorure de sodium, le sulfate ferreux, le sulfate ferrique, le sulfate de magnésium, le chlorure de calcium et le sulfate de manganèse ;
la vitamine est une ou plusieurs parmi la vitamine B1, la biotine et l'acide para-aminobenzoïque ;
de préférence, sur la base d'un milieu de fermentation de 1 l, la dose de source de carbone est de 20 à 60 g, plus préférentiellement de 40 à 50 g ; la dose de source d'azote organique est de 1 à 20 g, plus préférentiellement de 10 à 18 g ; la dose de source d'azote inorganique est de 0,1 à 10 g, plus préférentiellement de 0,5 à 5 g; la dose de sel inorganique est de 0 à 10 g, plus préférentiellement de 2,5 à 5 g ; la dose de vitamine est de 0 à 0,2 g, plus préférentiellement de 0,04 à 0,12 g.

9. Procédé selon la revendication 8, dans lequel sur la base d'un milieu de fermentation de 1 l, le milieu de fermentation contient 40 à 50 g de glucose, 8 à 12 g de peptone, 4 à 8 g d'extrait de bœuf, 0,6 à 1,2 g de sulfate d'ammonium, 0,25 à 0,75 g de chlorure de sodium, 0,05 à 0,2g de sulfate ferrique, 0,15 à 0,45 g de sulfate de magnésium, 0,05 à 0,15 g de chlorure de calcium, 0,5 à 4 g de dihydrogénophosphate de potassium, 1 à 4 g d'hydrogénophosphate de sodium, 0,01 à 0,06 g d'acide para-aminobenzoïque, 0,01 à 0,06 g de vitamine B1 et 0,001 à 0,006 g de biotine.

10. Procédé selon l'une quelconque des revendications 3 à 9, comprenant en outre : la réalisation d'une culture d'activation de souche et d'une culture de semences successivement avant fermentation, de préférence, le procédé de production de butanol comprend les étapes consistant à :
(1) inoculer la *C*. *beijerinckii* dans un milieu de culture solide, et cultiver entre 28 et 42 °C pendant 12 à 48 h dans une condition anaérobie ;
(2) inoculer la *C. beijerinckii* cultivée à l'étape (1) dans un milieu de culture de semences, et cultiver de manière statique entre 28 et 42 °C dans une condition anaérobie facultative pendant 12 à 48 h, afin d'obtenir une solution de semences ;
(3) inoculer la solution de semences obtenue à l'étape (2) dans une taille d'inoculum de 2 à 20% en volume dans un milieu de fermentation, et effectuer la fermentation dans une condition anaérobie facultative.

11. Procédé selon la revendication 4 ou 10, dans lequel la condition anaérobie facultative est créée par un procédé incluant : aucun désoxydant n'est ajouté dans le milieu de culture, et aucun N₂ et/ou gaz inerte n'est introduit pour maintenir un environnement anaérobie pendant la culture.
